# FASCICULE DE BREVET EUROPEEN

(11) **EP 0 649 309 B1**
(45) Date de publication et mention de la délivrance du brevet: **16.08.2001**
(21) Numéro de dépôt: 94916274.7
(22) Date de dépôt: 11.05.1994
(51) Int. Cl.: A61K 33/10, A61K 33/06, A61L 27/00

(54) **UTILISATION DE PARTICULES D'UN SEL DE CALCIUM BIOCOMPATIBLE ET BIORESORBABLE COMME INGREDIENT ACTIF DANS LA PREPARATION D'UN MEDICAMENT DESTINE AU TRAITEMENT LOCAL DES MALADIES DEMINERALISANTES DE L'OS**
VERWENDUNG VON TEILCHEN EINES BIOVERTRÄGLICHEN UND BIORESORBIERBAREN KALZIUMSALZES ALS WIRKSTOFF IN DER HERSTELLUNG EINES ARZNEIMITTELS ZUR LOKALEN BEHANDLUNG VON ENTKALKUNGSKRANKHEITEN
USE OF PARTICLES OF A BIOCOMPATIBLE, BIOABSORBABLE CALCIUM SALT AS ACTIVE PRINCIPLE IN THE PREPARATION OF A MEDICAMENT FOR LOCAL TREATMENT OF BONE DEMINERALIZING DISEASES

(30) Priorité: 13.05.1993 FR 9305783
(43) Date de publication de la demande: 26.04.1995
(73) Titulaire: BIO HOLDINGS INTERNATIONAL LIMITED, Tortola (VG)
(72) Inventeur: PATAT, Jean-Louis, F-75017 Paris (FR); CIROTTEAU, Yves, F-75007 Paris (FR)
(74) Mandataire: Hubert, Philippe
(86) Numéro de dépôt international: FR9400564
(87) Numéro de publication internationale: WO9426283

(56) Documents cités:
- EP-A- 0 395 187
- WO-A-86/01726
- WO-A-87/03491
- WO-A-89/06944
- DE-A- 4 130 546
- FR-A- 2 637 502

## Description

L'invention se rapporte au domaine du traitement de certaines maladies osseuses.

On sait que l'os est un tissu en perpétuel renouvellement. Schématiquement, des ostéoblastes sécrètent une matrice minérale constituée principalement d'hydroxyapatite, et l'ostéoblaste entouré de cette matrice minérale se transforme en ostéocyte. Concurremment, des cellules spécialisées, notamment des ostéoclastes, détruisent la matrice minérale. Dans l'os sain, l'espace ainsi libéré est occupé à nouveau par les ostéoblastes, et ainsi de suite.

Il est important de conserver un équilibre entre l'ostéogénèse et la résorption osseuse pour que l'os puisse assurer sa fonction dans le squelette.

On connaît un certain nombre de maladies liées à une déminéralisation ou à des troubles de minéralisation de l'os. La maladie la plus connue est certainement l'ostéoporose, dans laquelle le renouvellement du tissu osseux est déséquilibré car la destruction de la matrice osseuse par les ostéoclastes devient prépondérante. L'activité des ostéoblastes est fortement diminuée et à mesure de la progression de la maladie, la matrice minérale est présente en quantité insuffisante pour permettre à l'activité des ostéoblastes de se développer normalement. Il en résulte que la reconstruction de la matrice osseuse est interrompue ou en tout cas insuffisante pour maintenir les conditions nécessaires au processus d'ostéogénèse. Les ponts de tissu osseux de l'os spongieux sont réduits en nombre, en épaisseur et en volume relatif. La densité de l'os diminue, ce qui entraîne une fragilité mécanique plus grande. Il en résulte des fractures osseuses à la suite de chocs ou de chutes (notamment fractures du fémur, des poignets ou de l'humérus) ou encore des tassements vertébraux sous la pression exercée par le poids du corps. La réparation de telles fractures peut devenir difficile du fait de la faible masse osseuse disponible pour la réparation. On sait en particulier que les fractures du col du fémur chez les personnes âgées atteintes d'ostéoporose peuvent conduire à un décès dans environ 20 % des cas.

D'autres maladies sont accompagnées d'une déminéralisation ou d'une faible minéralisation ou d'un défaut de minéralisation de l'os. C'est le cas notamment de la maladie de Paget, des dysplasies fibreuses, et des ostéodystrophies provoquées notamment par certaines insuffisances rénales. En outre, la déminéralisation de l'os maxillaire peut être à l'origine de certaines maladies parodontales.

Jusqu'à présent les traitements des maladies déminéralisantes de l'os, en particulier de l'ostéoporose, comprennent un apport de calcium par voie orale, éventuellement en association avec la vitamine D, ou l'administration d'estrogènes éventuellement en combinaison avec des progestatifs. Ces traitements peuvent arrêter le processus de dégradation, sans permettre une reminéralisation par restauration de la trame osseuse.

L'administration de fluorures, également par voie orale, stimule l'activité ostéoblastique et peut entraîner une augmentation de la masse osseuse de 5 à 10 % par an chez les patients répondeurs (environ 60 % des cas). Toutefois, le traitement par les fluorures présente l'inconvénient d'une certaine toxicité par cumul des doses, avec apparition de microfissures osseuses qui peuvent être à l'origine de douleurs importantes.

On a maintenant découvert qu'il est possible de relancer la reminéralisation de l'os en apportant localement du calcium sous la forme d'un sel biocompatible et biorésorbable.

On a observé que l'application locale, à l'intérieur de l'os, d'un sel de calcium, permet de relancer un processus de reminéralisation et de restauration de la trame osseuse, avec notamment augmentation de la densité osseuse (et donc de la solidité de l'os), alors que l'apport de calcium par voie orale ne permet pas de relancer le processus de reconstruction des travées osseuses résorbées. En outre, grâce à ce traitement local, la reminéralisation est particulièrement rapide. Il convient de noter que le processus de reminéralisation s'étend à l'ensemble de l'os et non à la seule partie en contact avec la zone d'implantation du sel de calcium.

On a déjà décrit l'utilisation de divers sels de calcium, et en particulier de carbonate (notamment sous forme de corail) ou de phosphates (notamment sous la forme d'hydroxyapatite) comme implants utilisables comme pièces de prothèses osseuses ou de pièces de comblement favorisant la réhabitation par de l'os néo-formé, notamment dans le cas où le matériau de prothèse est biodégradable ou comporte un système de pores ouverts; voir notamment les documents EP-A-0 395 187, WO-A-8 906 944, FR-A-2 637 502, WO-A-8 601 726, DE-A-4 130 546 et WO-A-8 703 491.

L'invention a donc pour objet l'utilisation d'au moins un sel de calcium biocompatible et biorésorbable, sous forme de particules ayant des dimensions inférieures à 8 mm, comme ingrédient actif dans la préparation d'un médicament destiné au traitement local des maladies liées à la déminéralisation ou à des défauts de minéralisation de l'os, en particulier dans le but de rétablir le processus de reminéralisation de l'os.

Le médicament obtenu selon l'invention peut être utilisé notamment dans le traitement de l'ostéoporose, de la maladie de Paget, des dysplasies fibreuses et des ostéodystrophies. Il convient de noter en particulier que, dans le cas d'une ostéoporose, lorsqu'une fragilisation importante des os, et notamment du fémur, a été détectée, il est possible de traiter le malade avant qu'une fracture se produise.

Parmi les sels de calcium utilisables on citera en particulier le gluconate, le tartrate, le glycérophosphate, le sulfate, le carbonate et les phosphates, y compris le fluorophosphate, le phosphate tricalcique, etc.

Ces divers sels de calcium ont des temps de résorption différents, dépendant notamment de leur solubilité. On peut ainsi faire varier la vitesse de résorption par un mélange de sels de calcium ayant des vitesses de résorption différentes, ce qui permet à la fois un apport de calcium rapide tout en disposant d'une réserve pour un apport à long terme.

Lorsqu'ils sont présents, les phosphates, tels que le phosphate tricalcique ou l'hydroxyapatite, qui sont faiblement résorbables, sont employés de préférence en mélange avec d'autres sels de calcium plus rapidement résorbables. Selon un mode de réalisation particulier, on utilise un ingrédient actif exempt de phosphates.

Le matériau constituant l'ingrédient actif peut être présent notamment sous la forme de petites billes, de cylindres ou de granulés. Ces particules sont préparées selon les procédés usuels.

Généralement, elles ont des dimensions inférieures à 5 mm. Le plus souvent, on utilisera des particules ayant des dimensions de 0,2 à 4 mm, et notamment de 0,3 à 3 mm. La vitesse de résorption augmente lorsque les dimensions des particules diminuent.

Dans un mode de réalisation particulier, l'ingrédient actif du médicament obtenu selon l'invention contient au moins un carbonate de calcium, sous forme d'aragonite ou de calcite. Le carbonate peut être un matériau d'origine naturelle obtenu notamment à partir de squelettes de corail madréporaire (en particulier *Porites*, *Pocillopora*, *Acropora* ou *Favites*) ou de coquilles de mollusques (par exemple *Pinctada margaritifera*). Parmi les matériaux naturels à base de calcite, on citera en particulier les matériaux obtenus à partir de squelette d'échinoderme, notamment des épines d'oursin (tel que *Citaris*). Les traitements que l'on effectue par exemple sur le matériau de corail brut pour le transformer en matériau biocompatible (lavages, stérilisations) sont décrits notamment dans le document FR-2.460.657.

Les billes, cylindres ou granulés peuvent être obtenus notamment par broyage, par usinage et/ou par usure. Ils peuvent être constitués en matériau poreux ou non poreux.

Le médicament obtenu selon l'invention peut constituer une poudre administrable par injection (en particulier lorsqu'il s'agit de petites billes), à l'aide d'un dispositif injecteur muni d'un trocard. Dans le cas d'une opération à ciel ouvert, par exemple lors de la pose de pièces d'ostéosynthèse après une fracture du col fémoral, on peut directement mettre en place le médicament selon l'invention. Dans certains cas la mise en place à l'intérieur de l'os sera facilitée par mélange du sel de calcium avec un véhicule permettant de donner au médicament la consistance d'une pâte fluide. Le véhicule permettant de présenter le médicament sous forme de pâte fluide peut être du sérum physiologique, ou encore du sang, du plasma sanguin, de la moelle osseuse, ou une colle biologique. Ces véhicules d'origine naturelle peuvent être des produits autologues, c'est à dire prélevés sur l'individu traité lui-même ou obtenus à partir de substances prélevées sur l'individu traité.

On peut en outre mélanger les particules de sel de calcium ou les imprégner avec au moins une substance favorisant l'ostéogénèse, par exemple des sels de strontium (notamment le carbonate), des fluorures (par exemple fluorure de sodium), des facteurs de croissance, etc.

Le sang et la moelle osseuse notamment contiennent de tels facteurs de croissance.

On peut également préparer le médicament obtenu selon l'invention sous la forme d'une pâte fluide à l'aide d'une solution ou suspension de protéines comme par exemple le collagène, la fibronectine; etc, ou à l'aide d'un agent gélifiant biocompatible comme la carboxyméthyl cellulose ou la méthyl cellulose.

Dans le médicament obtenu selon l'invention, le sel de calcium représente généralement de 40 à 100 % en poids par rapport au poids total du médicament, et en particulier de 40 à 97 %.

Le médicament obtenu selon l'invention est administré, dans l'os spongieux et/ou dans le canal médullaire, de façon à appliquer une quantité efficace du sel de calcium, qui dépend du sel de calcium utilisé et de l'os à traiter (en particulier du volume de l'os) et de l'état du malade. Généralement, on administre de 1 à 25 g de calcium à l'aide du médicament obtenu selon l'invention. Dans le cas du traitement d'un fémur ou d'un humérus, on administre le médicament obtenu selon l'invention de façon à implanter par exemple de 2 à 20 g de calcium. Dans une vertèbre, on peut administrer par exemple une quantité de médicament suffisante pour apporter de 2 à 15 g de calcium.

Dans une forme de réalisation préférée, le médicament de l'invention contient des facteurs de croissance.

On sait que les facteurs de croissance sont des substances qui favorisent notamment la régénération des cellules et des tissus, et dont certaines ont été préconisées notamment dans le traitement de l'ostéoporose. Lors d'une lésion cellulaire *in vivo*, les thrombocytes (ou plaquettes) libèrent de nombreux facteurs de croissance, comme par exemple le PDGF (Platelet-derived growth factor), le TGF bêta (transforming growth factor bêta), les IGF-I et -II (insulin-related growth factor), etc.

On rappelle que l'on désigne par la dénomination "colles biologiques", des concentrés de protéines coagulables par la thrombine, ou des solutions de fibrinogène, qui sont utilisées dans la réalisation d'un matériau collant permettant notamment de réunir les tissus vivants tout en exerçant une action hémostatique ; voir par exemple le brevet FR-2.448.900. On peut préparer des colles biologiques enrichies en facteurs de croissance d'origine plaquettaire, par exemple selon le procédé de la demande de brevet FR n° 92.11643, déposée par la demanderesse le 30 Septembre 1992, ce procédé consistant principalement à congeler un plasma enrichi en plaquettes et à recueillir un cryoprécipité, constituant ladite colle, en réchauffant le plasma congelé à une température de +1 à +6°C environ, et en particulier vers +4°C.

Les exemples suivants illustrent l'invention.

### EXEMPLE 1 :

On a utilisé un premier médicament constitué par des billes de corail *Porites*, ayant des dimensions de 2 à 3 mm, commercialisées sous la dénomination BIO-SPHERE par la Société INOTEB.

On a aussi utilisé un second médicament constitué par des granulés de corail *Acropora*, ayant des dimensions de 0,3 à 1 mm, en mélange avec un gel de collagène, sous forme d'une pâte commercialisée sous la dénomination BIOCORAL GEL par la Société INOTEB. Ce produit contient 65 % en poids de granulés de corail.

On a utilisé ces médicaments pour traiter des patients âgés de plus de 80 ans, atteints d'ostéoporose se traduisant par un effondrement du corps vertébral au niveau lombaire.

A l'aide d'un dispositif injecteur muni d'un trocard, on a injecté dans le corps vertébral à traiter, par la voie postérieure et en passant par le pédicule, le médicament de l'invention.

Dans chaque cas, on a implanté, en une seule fois, environ 13 g de corail.

Au bout d'un mois, on peut observer (par radiographie) une reconstruction osseuse consolidant la vertèbre. Les douleurs consécutives au tassement vertébral s'atténuent puis disparaissent.

### EXEMPLE 2 :

On prend en charge des patients âgés de plus de 80 ans atteints d'ostéoporose, et souffrant d'une fracture du col fémoral.

Avant de mettre en place un système métallique clou-plaque d'ostéosynthèse, on introduit dans le foyer de fracture le médicament préparé à l'exemple 1, de façon à implanter dans chaque cas environ 20 g de corail.

Au bout d'un mois, on constate par exploration radiographique une reconstruction des travées osseuses. Au bout de trois mois, on observe une nette augmentation de l'épaisseur de la corticale de l'os traité. Au bout d'un an, l'exploration par tomodensitométrie montre que la masse osseuse est 8 fois supérieure à celle mesurée sur l'os opposé non traité.

Le patient peut être remis précocement en station debout.

### EXEMPLE 3 :

On procède sur des lapins à une trépanation bilatérale et symétrique du crâne au niveau des os pariétaux, par ablation de deux volets circulaires de 10 mm de diamètre.

On constitue cinq lots de lapins : quatre groupes de test et un groupe témoin. Sur les individus de chaque groupe de test, un seul côté est traité.

Groupe 1 (Symbole MF) : on applique dans l'une des fenêtres 50 microlitres de gel de méthylcellulose contenant 1 µg de TGF bêta.

Groupe 2 (Symbole BF) : on applique dans l'une des fenêtres 100 µl de colle biologique à laquelle on a ajouté 1 µg de TGF bêta.

Groupe 3 (Symbole BCF) : on applique 50 microlitres d'un mélange analogue à celui appliqué au groupe BF, mais contenant en outre 70 mg de granulés de corail *Porites*, commercialisés sous la dénomination BIOCORAL 450 par la Société INOTEB.

Groupe 4 (Symbole BC) : on applique le même mélange que pour le groupe 3, mais sans facteur de croissance.

On suit l'évolution de la construction osseuse par tomodensitométrie crânienne. Les diamètres et surface des trépanations sont mesurés sur un analyseur d'images. Après le sacrifice des animaux étudiés, au bout d'un mois, la calotte crânienne est prélevée puis préparée avec un microtome pour l'analyse histologique par histomorphométrie et microscopie en fluorescence. Pour marquer les surfaces d'ostéoformation, on a injecté 2 ml d'oxytétraquinol aux dixième et troisième jours précédant le sacrifice.

On a fait les observations suivantes :

Au bout de 30 jours, les groupes BC et BCF ont complètement recouvert la cicatrice par un tissu ostéoïde.

Pour les autres groupes, le recouvrement n'est pas complet. Le diamètre du côté traité est inférieur au diamètre du côté témoin. Le diamètre du côté témoin est inférieur aux diamètres observés dans le groupe témoin.

Le volume des travées osseuses (VTO) a été déterminé par histomorphométrie.

Le groupe BCF a le VTO le plus élevé, suivi par le groupe BC.

L'étude des animaux des groupes traités avec du corail montre que les granulés sont résorbés progressivement. Le corail a permis la création rapide de travées osseuses entre ces granulés.

D'une façon générale, l'adjonction du facteur de croissance élève la vitesse de reminéralisation.

## Revendications

1. Utilisation d'au moins un sel de calcium biocompatible et biorésorbable, sous forme de particules, ayant des dimensions inférieures à 8 mm, comme ingrédient actif dans la préparation d'un médicament destiné au traitement local des maladies liées à la déminéralisation ou à des défauts de minéralisation de l'os, dans le but de relancer le processus de reminéralisation de l'os.

2. Utilisation selon la revendication 1, caractérisée par le fait que lesdites maladies sont l'ostéoporose, la maladie de Paget, les dysplasies fibreuses et les ostéodystrophies.

3. Utilisation selon l'une quelconque des revendications précédentes caractérisée par le fait que que ledit médicament est destiné à être implanté dans la partie spongieuse ou dans le canal médullaire d'un os.

4. Utilisation selon l'une quelconque des revendications précédentes caractérisée par le fait que ledit sel de calcium est choisi parmi les suivants : le gluconate, le tartrate, le glycérophosphate, le sulfate, le carbonate et les phosphates.

5. Utilisation selon l'une quelconque des revendications précédentes, caractérisée par le fait que ledit sel de calcium est un carbonate.

6. Utilisation selon la revendication précédente, caractérisée par le fait que ledit carbonate est sous forme d'aragonite ou de calcite.

7. Utilisation selon la revendication précédente, caractérisée par le fait que ledit carbonate provient de squelettes de corail, de squelettes d'échinodermes, ou de coquilles de mollusques.

8. Utilisation selon l'une quelconque des revendications précédentes, caractérisée par le fait que ledit ingrédient actif est exempt de phosphates.

9. Utilisation selon l'une quelconque des revendications précédentes, caractérisée par le fait que lesdites particules ont des dimensions inférieures à 5 mm, et en particulier des dimensions pouvant aller de 0,2 à 4 mm.

10. Utilisation selon l'une quelconque des revendications précédentes caractérisée par le fait que lesdites particules sont mélangées avec au moins une substance favorisant l'ostéogénèse et/ou permettant de mettre lesdites particules sous la forme d'une pâte fluide.

11. Utilisation selon la revendication précédente, caractérisée par le fait que ladite substance est choisie parmi les sels de strontium, les fluorures, les facteurs de croissance, le sang, le plasma sanguin, la moelle osseuse, le collagène, une colle biologique, et un agent gélifiant biocompatible.

12. Utilisation selon l'une quelconque des revendications précédentes caractérisée par le fait que le sel de calcium représente de 40 à 100 % en poids par rapport au poids total du médicament.

## Patentansprüche

1. Verwendung mindestens eines biologisch verträglichen und resorbierbaren Calciumsalzes in Form von Teilchen mit Abmessungen von unter 8 mm als Wirkstoff bei der Herstellung eines Arzneimittels zur lokalen Behandlung von Krankheiten, die mit der Demineralisation oder mit Störungen der Mineralisation des Knochens verbunden sind, mit dem Ziel, den Vorgang der Remineralisation des Knochens auszulösen.

2. Verwendung nach Anspruch 1, **dadurch gekennzeichnet, dass** die Krankheiten Osteoporose, die Paget-Krankheit, fibröse Dysplasien und Osteodystrophien sind.

3. Verwendung nach einem der vorhergehenden Ansprüche, **dadurch gekennzeichnet, dass** das Arzneimittel vorgesehen ist, in die Substantia spongiosa oder den Rückenmarkskanal eines Knochens implantiert zu werden.

4. Verwendung nach einem der vorhergehenden Ansprüche, **dadurch gekennzeichnet, dass** das Calciumsalz aus Gluconat, Tartrat, Glycerinphosphat, Sulfat, Carbonat und den Phosphaten ausgewählt ist.

5. Verwendung nach einem der vorhergehenden Ansprüche, **dadurch gekennzeichnet, dass** das Calciumsalz ein Carbonat ist.

6. Verwendung nach dem vorhergehenden Anspruch , **dadurch gekennzeichnet, dass** das Carbonat in Form von Aragonit oder Calcit vorliegt.

7. Verwendung nach dem vorhergehenden Anspruch , **dadurch gekennzeichnet, dass** das Carbonat aus Korallenskeletten, Stachelhäuterskeletten oder Molluskenschalen stammt.

8. Verwendung nach einem der vorhergehenden Ansprüche, **dadurch gekennzeichnet, dass** der Wirkstoff phosphatfrei ist.

9. Verwendung nach einem der vorhergehenden Ansprüche, **dadurch gekennzeichnet, dass** die Abmessungen der Teilchen kleiner als 5 mm sind und insbesondere von 0,2 bis 4 mm reichen können.

10. Verwendung nach einem der vorhergehenden Ansprüche, **dadurch gekennzeichnet, dass** die Teilchen mit mindestens einer Substanz vermischt werden, welche die Knochenbildung begünstigt und/oder erlaubt, diese Teilchen in die Form einer fluiden Paste zu bringen.

11. Verwendung nach dem vorhergehenden Anspruch , **dadurch gekennzeichnet, dass** die Substanz aus Strontiumsalzen, Fluoriden, Wachstumsfaktoren, Blut, Blutplasma, Knochenmark, Collagen, einem biologischen Klebstoff und einem biologisch verträglichen Geliermittel ausgewählt ist.

12. Verwendung nach einem der vorhergehenden Ansprüche, **dadurch gekennzeichnet, dass** das Calciumsalz 40 bis 100 Gew.-% des Gesamtgewichts des Arzneimittels ausmacht.

## Claims

1. Use of at least one biocompatible and bioabsorbable calcium salt, in the form of particles, having dimensions less than 8 mm, as active ingredient in the preparation of a medicament intended for local treatment of diseases associated with demineralization or mineralization defects of bone, with the aim of reinitiating the process of bone remineralization.

2. Use according to Claim 1, characterized in that the said diseases are osteoporosis, Paget's disease, fibrous dysplasias and osteodystrophies.

3. Use according to any one of the preceding claims, characterized in that the said medicament is intended to be implanted in the spongy part or in the medullary canal of a bone.

4. Use according to any one of the preceding claims, characterized in that the said calcium salt is chosen from among the following: gluconate, tartarate, glycerophosphate, sulphate, carbonate and phosphates.

5. Use according to any one of the preceding claims, characterized in that the said calcium salt is a carbonate.

6. Use according to the preceding claim, characterized in that the said carbonate is in the form of aragonite or calcite.

7. Use according to the preceding claim, characterized in that the said carbonate comes from coral skeletons, echinoderm skeletons or mollusc shells.

8. Use according to any one of the preceding claims, characterized in that the active ingredient is phosphate-free.

9. Use according to any one of the preceding claims, characterized in that the said particles have dimensions less than 5 mm, and in particular dimensions which can range from 0.2 to 4 mm.

10. Use according to any one of the preceding claims, characterized in that the said particles are mixed with at least one substance promoting osteogenesis and/or allowing the said particles to be put into the form of a fluid paste.

11. Use according to the preceding claim, characterized in that the said substance is chosen from salts of strontium, fluorides, growth factors, blood, blood plasma, bone marrow, collagen, a biological adhesive and a biocompatible gelling agent.

12. Use according to any one of the preceding claims, characterized in that the calcium salt represents from 40 to 100 % by weight with respect to the total weight of the medicament.
